(19) Europäisches Patentamt
European Patent Office
Office européen des brevets

(11) Veröffentlichungsnummer: **0 212 535 B1**


(12) # EUROPÄISCHE PATENTSCHRIFT


(45) Veröffentlichungstag der Patentschrift: **29.05.91**

(51) Int. Cl.5: **C07D 473/34**, C07D 473/16, A61K 31/52

(21) Anmeldenummer: **86111116.9**

(22) Anmeldetag: **12.08.86**


(54) **N6-Disubstituierte Purinderivate, Verfahren zu deren Herstellung sowie diese Verbindungen enthaltende Arzneimittel.**


(30) Priorität: **17.08.85 DE 3529497**

(43) Veröffentlichungstag der Anmeldung:
**04.03.87 Patentblatt 87/10**

(45) Bekanntmachung des Hinweises auf die Patenterteilung:
**29.05.91 Patentblatt 91/22**

(84) Benannte Vertragsstaaten:
**AT BE CH DE FR GB IT LI LU NL SE**



(56) Entgegenhaltungen:

**EP-A- 155 911**
**CA-A- 522 947**
**CH-A- 551 996**
**DE-A- 2 249 027**
**US-A- 2 691 654**
**US-A- 3 813 394**
**US-A- 4 212 866**
**US-A- 4 294 831**
**US-A- 4 612 314**
**EP-A-01 386 83**
**CA-A- 778 787**
**CH-A- 551 997**
**JP-A-60 661 6**
**US-A- 3 016 378**
**US-A- 4 086 347**
**US-A- 4 241 063**
**US-A- 4 609 661**

**J.Am. Chem. Soc. 83, 2574-79 (1961)**

**Chem. Abstr. 54, 9935 (1960)**



(73) Patentinhaber: **BOEHRINGER MANNHEIM GMBH**
**Sandhofer Strasse 116**
**W-6800 Mannheim 31(DE)**

(72) Erfinder: **Friebe, Walter Gunar, Dr. rer. nat.**
**Sophienstrasse 8**
**W-6800 Mannheim(DE)**
Erfinder: **Wilhelms, Otto-Henning, Dr. rer. nat.**
**Odenwaldstrasse 25/2**
**W-6941 Weinheim-Rittenweier(DE)**





Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

Chem. Abstr. 56, 11588-89 (1962)

Chem. Abstr. 60, 4401-03 (1960)

Chem. Abstract 63, 16350 f,g (1965)

Chem. Abstract 64, 3911 c,d (1960)

Chem. Abstract 85, 46563 u (1976)

Chem. Abstract 107, 115913 v (1987)

Austr. J. Chem. 22, 205-19 (1969)

Chem. Abstr. 63, 13025e ( = J. Het. Chem. 2, (1965), 291-7)

Chem. Abstr. 65, 2072 d ( = US-A-3,251,738)

Chem. Abstr. 57, 13780 i ( =US-A-3,041,340)

## Beschreibung

Die vorliegende Erfindung betrifft neue $N^6$-disubstituierte Purinderivate, Verfahren zu deren Herstellung sowie diese Verbindungen enthaltende Arzneimittel.

Die erfindungsgemaeßen Verbindungen koennen die antigenbedingte SRS-A-Freisetzung aus Lungengewebeproben hemmen. Sie eignen sich daher zur Behandlung allergischer Krankheiten sowie von entzuendungsbedingten bronchospastischen und bronchokonstriktorischen Reaktionen.

Die vorliegende Erfindung betrifft $N^6$-disubstituierte Purinderivate der allgemeinen Formel I

(I),

in welcher

$R_1$ und $R_4$ gleich oder verschieden sind und jeweils Wasserstoff, ein Halogenatom oder eine Gruppe $-NR_6R_7$ bedeuten,

$R_2$ und $R_3$ gleich oder verschieden sind und jeweils einen $C_1$- bis $C_6$-Alkylrest, einen $C_2$- bis $C_6$-Alkenylrest, einen $C_3$- bis $C_7$-Cycloalkylrest, einen gewuenschtenfalls am Phenylteil ein- oder zweifach durch Halogen, Hydroxy, $C_1$- bis $C_6$-Alkyl, $C_1$- bis $C_6$-Alkoxy, $C_1$- bis $C_6$-Alkylthio oder Trifluormethyl substituierten geradkettigen oder verzweigten Phenalkylrest mit 1 bis 4 Kohlenstoffatomen im Alkylteil, einen Furylmethyl- oder Furylethylrest, einen Thiophenmethyl- oder Thiophenethylrest, einen gewuenschtenfalls durch einen $C_1$- bis $C_4$-Alkylrest substituierten Pyridylmethylrest oder Pyridylethylrest, einen Indanylrest oder einen Tetrahydronaphthylrest,

$R_5$ Wasserstoff, Tetrahydrofuranyl, Tetrahydropyranyl oder einen $C_1$- bis $C_{10}$-Alkylrest, der gewuenschtenfalls ein- oder mehrfach durch Hydroxy, $C_1$- bis $C_6$-Alkanoyloxy, $C_1$- bis $C_6$-Alkoxycarbonyl, $C_2$- bis $C_6$-Carboxyalkanoyloxy, $C_2$- bis $C_6$-Hydroxyalkoxy, $C_3$-bis $C_6$-Dihydroxyalkoxy, Carboxy, Cyano oder 1H-Tetrazol-5-yl substituiert sein kann,

$R_6$ und $R_7$ die gleich oder verschieden sind, jeweils Wasserstoff, einen $C_1$- bis $C_6$-Alkylrest oder auch gemeinsam einen gewuenschtenfalls durch Sauerstoff, Schwefel oder eine Gruppe $NR_8$ unterbrochenen $C_2$- bis $C_5$-Alkylenrest und

$R_8$ Wasserstoff oder einen $C_1$- bis $C_6$-Alkylrest bedeuten,
mit der Maßgabe, daß

a) $R_2$ und $R_3$ nicht gleichzeitig einen $C_1$-$C_6$-Alkylrest bedeuten können, oder

b) $R_2$ und $R_3$ nicht gleichzeitig ein $C_2$-$C_6$-Alkenylrest, eine unsubstituierte Benzyl- oder Furylmethylgruppe sein können, wenn $R_5$ ein Wasserstoffatom ist, oder

c) $R_2$ nicht eine $C_2$-$C_6$-Alkenylgruppe, eine unsubstituierte Benzyl- oder Furylmethylgruppe sein kann, wenn $R_3$ eine Methylgruppe und $R_5$ ein Wasserstoffatom darstellt,

deren pharmakologisch verträgliche Salze, sowie Verfahren zu deren Herstellung.

Weiterhin betrifft die Erfindung pharmazeutische Präparate mit einem Gehalt an Verbindungen der Formel I sowie die Verwendung dieser Verbindungen zur Herstellung solcher Präparate.

Aus dem Stand der Technik sind einige $N^6$-disubstituierte Purinderivate bereits bekannt. CH-A-551,966 und CH-A-551,997 beschreiben Verbindungen, bei denen die beiden Substituenten $R_2$ und $R_3$ am Stickstoffatom gleichzeitig einen Alkylrest bedeuten. Die übrigen in diesen beiden Patentschriften genannten Verbindungen betreffen am Stickstoffatom monosubstituierte Reste, wie z.B. Phenylalkyl- und Acyl-Derivate. EP-A-155,911 beschreibt unter anderem ebenfalls Verbindungen mit zweifach substituiertem Stickstoffatom in 6-Stellung des Purinringes, sowie Phenyl- und Naphthylgruppen, die direkt an das $N^6$-Stickstoffatom des Adeninrestes gebunden sind. Die vorliegende Erfindung schließt derartige Verbindungen nicht ein, da $R_2$ und $R_3$ nicht gleichzeitig eine $C_1$-$C_6$-Alkylgruppe sein können, bzw. als Substituenten nicht die Phenylgruppe, sondern die Phenylalkylgruppen beansprucht werden. Analoge Verbindungen sind außerdem in den folgenden Dokumenten beschrieben: US-A-2,691,654; US-A-3,016,378; US-A-3,813,394; US-A-4,294,831; US-A-4,609,661; US-A-4,612,314; US-A-4,086,347; US-A-4,212,866; US-A-4,241,063; CA-A-522,947; DE-A-2,249,027; JP-60/6616; EP-A-0,138,683; J. Am. Chem. Soc. 83, 2574-79 (1961); Chem. Abstr. 54, 9935 (1960); Chem. Abstr. 56, 11588-89 (1962); Chem. Abstr. 60, 4401-03 (1960); Chem. Abstr. 63, 16350 f,g (1965); Chem. Abstr. 64, 3911 c,d (1960); Chem. Abstr. 85, 46563 u (1976); Chem. Abstr. 107, 115913 v (1987).

Verbindungen der Formel I, in denen $R^2$ und $R^3$ gleichzeitig einen $C_2$-$C_6$-Alkenylrest und $R^5$ ein Wasserstoffatom bedeutet, sind nicht Gegenstand der Erfindung. Derartige Derivate sind beschrieben in CA-A-778,787 (6-N-(gamma,gamma-Dimethylallyl)amino-purin); Austr. J. Chem. 22, 205-19 (1969) (6-N-Diallylamino-purin). Verbindungen mit $R^2 = R^3$ = unsubstituiertes Benzyl und $R^5 = H$ werden in Chem. Abstr. 63, 13025 e (= J. Het. Chem. 2, (1965), 291-7) beschrieben. Die Verbindung mit $R^1 = R^4 = R^5 = H$ und $R^2 = R^3$ = Furylmethyl (= Furfurylkinetin) ist aus Chem. Abstr. 65, 2072 d (= US-A-3,251,738) bekannt. Die zuvor genannten Purin-Derivate werden durch Disclaimer b) von der Erfindung ausgenommen.

Verbindungen der Formel I, in denen $R^1 = R^4 = R^5 = H$, $R^2 = CH_3$ und $R^3$ = Furylmethyl oder Benzyl bedeutet, sind in Chem. Abstr. 57, 13780 i (= US-A-3,041,340) beschrieben. N-Allyl-N-methylaminopurin ist aus Austr. J. Chem. 1969, 22 (1), 205-219 bekannt. Derartige Verbindungen werden durch Disclaimer c) von der Erfindung ausgenommen.

Verbindungen der Formel I sind insbesondere Purinderivate, in denen

$R_1$ und $R_4$ gleich oder verschieden sind und jeweils Wasserstofff, eine Methylamino- oder Dimethylamino-Gruppe bedeuten,

$R_2$ und $R_3$ gleich oder verschieden sind und jeweils einen 2-Propyl-, 2-Methylpropyl-, 3-Pentyl-, Allyl-, Cyclopentyl-, Cyclohexyl-, Indanyl-, einen ggf. ein- oder zweifach durch Chlor, Fluor, Methyl, Trifluormethyl oder Methoxy substituierten Benzylrest, eine Furylmethyl-, Thiophenmethyl-, Thiophenethylgruppe oder eine ggf. durch Methyl substituierte Pyridinmethylgruppe und

$R_5$ Wasserstoff, Tetrahydrofuranyl, Tetrahydropyranyl oder einen 2,3-Dihydroxypropyl-, 1-(1,3-dihydroxy-2-propoxy)-2-hydroxy-ethyl-, 2-Hydroxypropyl-, 2-Acetoxypropyl-, 2-Succinoyloxy-propyl-, 8-Carboxyoctyl-, 4-Cyanobutyl-, (2-Hydroxyethoxy)methyl oder 4-(1H-tetrazolyl)butylrest

bedeuten, sowie deren pharmakologisch verträgliche Salze.

Die Alkylreste in den genannten Gruppen sowie die Alkenyl-, die Alkoxy- und die Alkylthioreste können geradkettig oder verzweigt sein. Bevorzugte Alkylreste sind der Methyl-, Ethyl-, n-Propyl-, Isopropyl-, n-Butyl-, Isobutyl-, n-Pentyl- und 3-Pentylrest. Ein Alkenylrest ist insbesondere der Allylrest. Alkoxy- und Alkylthioreste sind vorzugsweise der Methoxy-, Ethoxy-, Methylthio- und Ethylthio-rest.

Als Phenalkyl kommen beispielsweise der Benzyl-, Phenethyl-, Phenylpropyl-, Phenylisopropyl- oder 3-Methyl-3-phenyl-propyl-rest in Frage.

Für den Rest $R_5$ können insbesondere folgende Gruppen stehen: Wasserstoff, Tetrahydrofuran-2-yl, Tetrahydropyran-2-yl, 2-Hydroxy-propyl, 2,3-Dihydroxy-propyl, 2-Acetoxypropyl, 2-Succinoyloxypropyl, (2-Hydroxyethoxy)methyl, 1-(1,3-Dihydroxy-2-propoxy)-2-hydroxy-ethyl, 8-Methoxycarbonyl-octyl, 8-Carboxyoctyl, 4-Cyanobutyl und 4-(1H-Tetrazol-5-yl)butyl. Halogenatome sind insbesondere Fluor, Chlor und Brom.

Außer den in den Beispielen genannten Verbindungen sind Gegenstand der vorliegenden Erfindung insbesondere alle Substanzen, die jede moegliche Kombination der in den Beispielen genannten Substituenten aufweisen.

Das erfindungsgemaeße Verfahren zur Herstellung der Verbindungen der Formel I ist dadurch gekennzeichnet, daß man in an sich bekannter Weise eine Verbindung der allgemeinen Formel II

(II),

in welcher

$R_1$ und $R_4$ die obengenannte Bedeutung haben,

X einen reaktiven Rest darstellt und

A fuer die obengenannte Gruppe $R_5$ oder einen Ribofuranosylrest steht,

mit einer Verbindung der allgemeinen Formel III

$HNR_2R_3$ (III),

in welcher
$R_2$ und $R_3$ die obengenannte Bedeutung haben, umsetzt
und anschließend gewuenschtenfalls
einen fuer A stehenden Ribofuranosylrest in einen Rest $R_5$ umwandelt oder durch einen Rest $R_5$ ersetzt,
eine in $R_5$ enthaltene Hydroxylgruppe durch Abspaltung einer Schutzgruppe freilegt,
eine in $R_5$ enthaltene Hydroxylgruppe durch Umsetzung mit einem reaktiven Carbonsaeurederivat verestert,
eine in $R_5$ enthaltene Alkoxycarbonylgruppe zur Carboxylgruppe verseift,
eine in $R_5$ enthaltene Cyanogruppe durch Umsetzung mit Stickstoffwasserstoffsaeure in eine 1H-Tetrazol-5-ylgruppe ueberfuehrt,
einen fuer $R_5$ stehenden Tetrahydrofuranyl- oder Tetrahydropyranyl-rest durch Wasserstoff ersetzt,
ein fuer $R_5$ stehendes Wasserstoffatom durch einen anderen, durch die Definition von $R_5$ gegebenen Rest ersetzt,
eine Gruppe $R_1$ oder $R_4$ in eine andere, durch die Definition von $R_1$ und $R_4$ gegebene Gruppe ueberfuehrt,
und die erhaltenen Verbindungen der Formel I gegebenenfalls durch Neutralisation mit nichttoxischen Saeuren in ihre pharmakologisch vertraeglichen Salze umwandelt.

Als reaktive Reste X kommen Chlor, Brom und Niederalkylthio in Frage.

Eine Umwandlung eines Euer A stehenden Ribofuranosylrestes in einen Rest $R_5$ kann beispielsweise durch Behandlung mit Natriummetaperiodat und anschließende Reduktion mit Natriumtetrahydridoboranat erfolgen.

Desweiteren kann ein Ribofuranosylrest durch Behandlung mit einer Mineralsaeure abgespalten werden und das erhaltene Produkt mit $R_5$ gleich Wasserstoff durch Alkylierung in ein Derivat der Formel I, in dem $R_5$ verschieden von Wasserstoff ist, ueberfuehrt werden.

Die Abspaltung einer Hydroxylschutzgruppe kann entsprechend ihrer Eigenart entweder
im Fall von Alkanoyl- oder Aroylresten durch saure oder alkalische Verseifung oder
im Fall von Benzylschutzgruppen durch Hydrogenolyse oder Behandlung mit Mineralsaeuren oder
im Fall von Ketalschutzgrupen durch saure Hydrolyse erfolgen.

Fuer die Veresterung von Hydroxylgruppen kommen Carbonsaeuren oder reaktive Derivate hiervon, wie beispielsweise Carbonsaeurehalogenide oder -anhydride, Carbonsaeureazide oder aktivierte Ester in Frage.

Die Umsetzung einer Cyanogruppe mit Stickstoffwasserstoffsaeure erfolgt zweckmaeßig durch Erzeugung der letzteren in situ, beispielweise aus einem Alkaliazid und Ammoniumchlorid.

Fuer die Abspaltung eines Tetrahydrofuranyl- oder Tetrahydropyranylrestes eigenen sich anorganische Saeuren wie Salzsaeure oder Schwefelsaeure in waessriger oder organischer Loesung.

Eine Umwandlung von Verbindungen der Formel I, in denen $R_5$ Wasserstoff bedeutet, in Verbindungen der Formel I, in denen $R_5$ verschieden von Wasserstoff ist, erfolgt zweckmaeßig durch Alkylierung mit einer Verbindung $R_5$-Y, worin Y einen reaktiven Rest wie beispielsweise Halogen, Methansulfonyloxy oder Toluolsulfonyloxy bedeutet, in einem saeurebindenden Milieu.

Eine Umwandlung eines Restes $R_1$ oder $R_4$ in einen anderen, durch die Definition gegebenen Rest $R_1$ oder $R_4$ kann beispielsweise erfolgen durch
Ersatz eines Wasserstoffatoms durch Halogenierung, Ersatz eines Halogenatoms durch Aminierung, Austausch einer Aminofunktion gegen Halogen durch Umsetzung mit einem Nitrosylhalogenid.

Die Ausgangsverbindungen der allgemeinen Formel II und III sind literaturbekannte Substanzen oder koennen in Analogie zu literaturbekannten Verfahren hergestellt werden.

Die pharmakologisch vertraeglichen Salze erhaelt man in ueblicher Weise, z.B. durch Neutralisation der Verbindungen der Formel I mit nichttoxischen anorganischen oder organischen Saeuren wie z.B. Salzsaeure, Schwefelsaeure, Phosphorsaeure, Bromwasserstoffsaeure, Essigsaeure, Milchsaeure, Zitronensaeure, Aepfelsaeure, Salicylsaeure, Malonsaeure, Maleinsaeure oder Bernsteinsaeure.

Zur Herstellung von Arzneimitteln werden die Verbindungen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die Substanzen der allgemeinen Formel I koennen in fluessiger oder fester Form oral und parenteral appliziert werden. Als Injektionsmedium kommt vorzugsweise Wasser zur Anwendung, welches die bei Injektionsloesungen ueblichen Stabilisierungsmittel, Loesungsvermittler und/oder Puffer enthaelt. Derartige Zusaetze sind z.B. Tartrat- oder Borat-Puffer, Ethanol, Dimethylsulfoxid, Komplexbildner (wie Ethylendiamintetraessigsaeure), hochmolekulare Polymere (wie fluessiges Polyethylenoxid) zur Viskositaetsregulierung

oder Polyethylen-Derivate von Sorbitanhydriden.

Feste Traegerstoffe sind z.B. Staerke, Lactose, Mannit, Methylcellulose, Talkum, hochdisperse Kieselsaeure, hoehermolekulare Polymere (wie Polyethylenglykole).

Fuer die orale Applikation geeignete Zubereitungen koennen gewuenschtenfalls Geschmacks- und Sueßstoffe enthalten. Fuer die aeußerliche Anwendung koennen die erfindungsgemaeßen Substanzen I auch in Form von Pudern und Salben verwendet werden. Sie werden dazu z.B. mit pulverfoermigen, physiologisch vertraeglichen Verduennungsmittel bzw. ueblichen Salbengrundlagen vermischt.

Die verabreichte Dosierung haengt vom Alter, der Gesundheit und dem Gewicht des Empfaengers, dem Ausmaß der Krankheit, der Art gleichzeitiger gegebenenfalls durchgefuehrter weiterer Behandlungen, der Haeufigkeit der Behandlungen und der Art der gewuenschten Wirkung ab. Ueblicherweise betraegt die taegliche Dosis der aktiven Verbindung 0.1 bis 50 mg/kg Koepergewicht. Normalerweise sind 0.5 bis 40 und vorzugsweise 1.0 bis 20 mg/ kg/Tag in einer oder mehreren Anwendungen pro Tag wirksam, um die gewuenschten Resultate zu erhalten.

## Beispiel 1

### 6-[N-Benzyl-N-(2-methyl-propyl)amino]-9-tetrahydropyran-2-yl-purin

Eine Mischung aus 10.7 g (40 mmol) 6-Chlor-9-tetrahydropyran-2-yl-purin, 19.5 g (120 mmol) N-Benzyl-2-methylpropylamin und 60 ml Butanol wird 16 h zum Rueckfluß erhitzt. Man engt ein, nimmt den Rueckstand in Dichlormethan auf, waescht mit verd. Essigsaeure und anschließend mit Wasser, trocknet ueber Natriumsulfat, engt ein und verreibt mit Ligroin.
Es verbleiben 14.2 g Titelverbindung (97 % d.Th.) vom Schmp. 93-95 °C.

## Beispiel 2

In analoger Weise wie in Beispiel 1 beschrieben erhaelt man:

| | Bezeichnung | Ausbeute [%] | Schmelzpunkt°C Lsg.-mittel |
|---|---|---|---|
| a) | 6-[N-(5-Chlor-2-methoxy)benzyl-N-cyclopentyl-amino]-9-tetrahydropyran-2-yl-purin aus 6-Chlor-9-tetrahydropyran-2-yl-purin und N-(5-Chlor-2-methoxy)benzyl-cyclopentylamin | 75 | 142-144 (Ligroin) |
| b) | 6-[N-Cyclopentyl-N-(furan-2-yl-methyl)-amino]-9-tetrahydropyran-2-yl-purin aus 6-Chlor-9-tetrahydropyran-2-yl-purin und N-Cyclopentyl-(furan-2-yl-methyl)-amin | 85 | Hydrochlorid 143-144 (Diethylether) |
| c) | 6-(N-Allyl-N-cyclohexyl-amino)-9-tetrahydrofuran-2-yl-purin aus 6-Chlor-9-tetrahydrofuran-2-yl-purin und N-Allyl-cyclohexylamin | 54 | Oel (Dichlormethan) |
| d) | 6-(N-Allyl-N-cyclohexyl-amino)-9-tetrahydropyran-2-yl-purin aus 6-Chlor-9-tetrahydropyran-2-yl-purin-und N-Allyl-cyclohexylamin | 69 | 77-78 (Hexan) |
| e) | 6-[N-Cyclopentyl-N-(3-trifluormethyl-benzyl)amino]-9-tetrahydropyran-2-yl-purin aus 6-Chlor-9-tetrahydropyran-2-yl-purin und N-Cyclopentyl-(3-trifluormethyl-benzyl)amin | 52 | 112-113 (Ligroin) |

Beispiel 2 ( Fortsetzung )

| | Bezeichnung | Ausbeute [%] | Schmelzpunkt°C Lsg.-mittel |
|---|---|---|---|
| f) | 6-(N-Benzyl-N-cyclopentyl-amino)-9-tetrahydrofuran-2-yl-purin aus 6-Chlor-9-tetrahydrofuran-2-yl-purin und N-Benzyl-cyclopentylamin | 67 | 88-90 (Hexan) |
| g) | 6-(N-Benzyl-N-cyclopentyl-amino)-9-tetrahydropyran-2-yl-purin aus 6-Chlor-9-tetrahydropyran-2-yl-purin und N-Benzyl-cyclopentylamin | 71 | 91-93 (Hexan) |
| h) | 6-[N-Cyclopentyl-N-(4-methoxy-benzyl)-amino]-9-tetrahydrofuran-2-yl-purin aus 6-Chlor-9-tetrahydrofuran-2-yl-purin und N-Cyclopentyl-(4-methoxy-benzyl)-amin | 83 | 74-76 (Hexan) |
| i) | 6-[N-Cyclopentyl-N-(4-methoxy-benzyl)-amino]-9-tetrahydropyran-2-yl-purin aus 6-Chlor-9-tetrahydropyran-2-yl-purin und N-Cyclopentyl-(4-methoxy-benzyl)-amin | 66 | 97-99 (Ligroin) |
| j) | 6-[N-Cyclopentyl-N-(2,5-dimethyl-benzyl)amino]-9-tetrahydrofuran-2-yl-purin aus 6-Chlor-9-tetrahydrofuran-2-yl-purin und N-Cyclopentyl-(2,5-dimethyl-benzyl)amin | 48 | Oel (Dichlormethan) |
| k) | 6-[N-Cyclopentyl-N-(2,5-dimethyl-benzyl)amino]-9-tetrahydropyran-2-yl-purin aus 6-Chlor-9-tetrahydropyran-2-yl-purin und N-Cyclopentyl-(2,5-dimethyl-benzyl)amin | 51 | 103-105 (Ligroin) |

Beispiel 2 ( Fortsetzung)

| | Bezeichnung | Ausbeute [%] | Schmelzpunkt°C Lsg.-mittel |
|---|---|---|---|
| l) | 6-[N-Cyclopentyl-N-(thiophen-2-yl-methyl)-amino]-9-tetrahydrofuran-2-yl-purin aus 6-Chlor-9-tetrahydrofuran-2-yl-purin und N-Cyclopentyl-(thiophen-2-yl-methyl) amin | 56 | 62-64 (Hexan) |
| m) | 6-[N-Cyclopentyl-N-(thiophen-2-yl-methyl)-amino]-9-tetrahydropyran-2-yl-purin aus 6-Chlor-9-tetrahydropyran-2-yl-purin und N-Cyclopentyl-(thiophen-2-yl-methyl)-amin | 79 | 74-76 (Ligroin) |
| n) | 6-<N-(2-Methyl-propyl)-N-[2-(thiophen-2-yl)ethyl]amino>-9-tetrahydropyran-2-yl-purin aus 6-Chlor-9-tetrahydropyran-2-yl-purin und N-(2-Methyl-propyl)-[2-(thiophen-2-yl)ethyl]amin | 70 | Oel (Dichlormethan) |
| o) | 6-[N-(Indan-1-yl)-N-(2-methyl-propyl)-amino]-9-tetrahydropyran-2-yl-purin aus 6-Chlor-9-tetrahydropyran-2-yl-purin und N-(Indan-1-yl)-(2-methyl-propyl)-amin | 63 | 108-110 (Ligroin) |
| p) | 6-[N-Cyclopentyl-N-(4-methyl-benzyl)-amino]-9-tetrahydropyran-2-yl-purin aus 6-Chlor-9-tetrahydropyran-2-yl-purin und N-Cyclopentyl-(4-methyl-benzyl)-amin | 92 | Oel (Dichlormethan) |

Beispiel 2 (Fortsetzung)

| | Bezeichnung | Ausbeute [%] | Schmelzpunkt°C Lsg.-mittel |
|---|---|---|---|
| q) | 6-[N-(4-Chlor-benzyl)-N-(3-pentyl)-amino]-9-tetrahydropyran-2-yl-purin aus 6-Chlor-9-tetrahydropyran-2-yl-purin und N-(4-Chlor-benzyl)-3-pentylamin | - 77 | Oel (Dichlormethan) |
| r) | 6-[N-Allyl-N-(indan-1-yl)amino]-9-tetrahydropyran-2-yl-purin aus 6-Chlor-9-tetrahydropyran-2-yl-purin und N-Allyl-indan-1-yl-amin | 75 | Hydrochlorid 155-160 (Diethylether) |
| s) | 6-[N-(4-Methoxy-benzyl)-N-(3-pentyl)-amino]-9-tetrahydropyran-2-yl-purin aus 6-Chlor-9-tetrahydropyran-2-yl-purin und N-(4-Methoxy-benzyl)-3-pentyl-amin | 86 | 115-118 (Diethylether) |
| t) | 6-[N-Cyclopentyl-N-(2-methyl-benzyl)-amino]-9-tetrahydropyran-2-yl-purin aus 6-Chlor-9-tetrahydropyran-2-yl-purin und N-Cyclopentyl-(2-methyl-benzyl)-amin | 90 | Oel (Diethylether) |
| u) | 6-[N-Cyclopentyl-N-(pyridin-2-yl-methyl)amino]-9-tetrahydropyran-2-yl-purin aus 6-Chlor-9-tetrahydropyran-2-yl-purin und N-Cyclopentyl-N-(pyridin-2-yl-methyl)amin | 62 | Oel (Dichlormethan) |
| v) | 6-[N-Cyclopentyl-N-(2-methyl-pyridin-6-yl-methyl)amino]-9-tetrahydropyran-2-yl-purin aus 6-Chlor-9-tetrahydropyran-2-yl-purin und N-Cyclopentyl-N-(2-methyl-pyridin-6-yl-methyl)amin | 66 | 126-129 (Diethylether) |

Beispiel 3

6-[N-Cyclopentyl-N-(2,5-dimethyl-benzyl)amino]-9-[(2-hydroxyethoxy)methyl]-purin-hydrochlorid

Eine Mischung aus 9.0 g (27 mmol) 9-[(2-Benzoyloxy-ethoxy) methyl]-6-chlor-purin, 16.5 g (81 mmol) N-Cyclopentyl-(2,5-dimethyl-benzyl)amin und 130 ml Butanol wird 16 h zum Rueckfluß erhitzt. Man engt ein, nimmt den Rueckstand in Dichlormethan auf, waescht mit verd. Essigsaeure und anschließend mit Wasser, trocknet ueber Natriumsulfat, engt ein, versetzt mit der Loesung von 3.3 g Natrium in 200 ml Methanol, erhitzt 3 h zum Rueckfluß, engt ein, nimmt in Wasser auf, extrahiert mit Dichlormethan, trocknet

ueber Natriumsulfat und engt ein. Nach Chromatographie an Kieselgel mit einer Elutionsmischung aus 95 % Dichlormethan und 5 % Methanol erhaelt man 8.0 g Titelverbindung (75 % d-Th.) als oelige Base, deren in Ethylacetat hergestelltes Hydrochlorid bei 132-135 °C schmilzt.

Beispiel 4

In analoger Weise wie in Beispiel 3 beschrieben erhaelt man: 6-[N-Cyclopentyl-N-(thiophen-2-yl-methyl)amino]-9-[(2-hydroxyethoxy)methyl]-purin
in 78 % Ausbeute als oelige Base.

Beispiel 5

6-[N-Benzyl-N-(2-methyl-propyl)amino]purin

Eine Loesung aus 17.0 g (47 mmol) 6-[N-Benzyl-N-(2-methylpropyl)amino]-9-tetrahydropyran-2-yl-purin (Verbindung des Beispiels 1) in 200 ml gesaettigter ethanolischer Chlorwasserstoffloesung wird 10 min zum Rueckfluß erhitzt. Nach Stehen ueber Nacht verduennt man mit Wasser, stellt mit Ammoniakwasser auf pH 7 ein und saugt den Niederschlag ab. Nach Umkristallisation aus waessrigem 2-Propanol verbleiben 10.5 g Titelverbindung (80 % d.Th.) vom Schmelzpunkt 160-162 °C.

Beispiel 6

In analoger Weise wie in Beispiel 5 beschrieben erhaelt man:

| | Bezeichnung | Ausbeute [%] | Schmelzpunkt°C Lsg.-mittel |
|---|---|---|---|
| a) | 6-[N-Cyclopentyl-N-(3-trifluor-methyl-benzyl)amino]purin aus Verbindung des Beispiel 2 e | 72 | 163-165 (2-Propanol) |
| b) | 6-[N-(5-Chlor-2-methoxy)benzyl-N-cyclopentyl-amino]purin aus Verbindung des Beispiel 2 a | 63 | 219-221 (2-Propanol) |

Beispiel 6 ( Fortsetzung )

| | Bezeichnung | Ausbeute [%] | Schmelzpunkt°C Lsg.-mittel |
|---|---|---|---|
| c) | 6-[N-Cyclopentyl-N-(4-methyl-benzyl)-amino]purin aus Verbindung des Beispiel 2 p | 61 | 220-222 (Wasser) |
| d) | 6-[N-(4-Chlor-benzyl)-N-(3-pentyl)-amino]purin aus Verbindung des Beispiel 2 q | 82 | 153-155 (Ligroin) |
| e) | 6-<N-(2-Methyl-propyl)-N-[2-(thiophen-2-yl)ethyl]amino>purin aus Verbindung des Beispiel 2 n | 85 | 115-117 (Ligroin) |
| f) | 6-[N-(Indan-1-yl)-N-(2-methyl-propyl)-amino]purin aus Verbindung des Beispiel 2 o | 43 | 86-88 (Diethylether) |
| g) | 6-[N-Allyl-N-(indan-1-yl)amino]-purin aus Verbindung des Beispiels 2 r | 91 | Hydrochlorid 88-90 (Ethanol) |
| h) | 6-[N-(4-Methoxy-benzyl)-N-(3-pentyl)-amino]purin aus Verbindung des Beispiel 2 s | 53 | 152-155 (Diethylether) |
| i) | 6-[N-Cyclopentyl-N-(2-methyl-benzyl)-amino]purin aus Verbindung des Beispiel 2 t | 83 | 190-193 (Wasser) |
| j) | 6-[N-Cyclopentyl-N-(thiophen-2-yl-methyl)amino]purin aus Verbindung des Beispiel 2 m | 74 | 182-184 (Diethylether) |
| k) | 6-[N-Cyclopentyl-N-(4-methoxy-benzyl)-amino]purin aus Verbindung des Beispiel 2 i | 47 | 170-172 (Wasser) |

Beispiel 6 (Fortsetzung)

| | Bezeichnung | Ausbeute [%] | Schmelzpunkt°C Lsg.-mittel |
|---|---|---|---|
| l) | 6-[N-Cyclopentyl-N-(3-trifluormethyl-benzyl)amino]-2-methylamino-purin aus Verbindung des Beispiels 23 b | 39 | 80-90 (amorph) (Dichlormethan) |
| m) | 6-[N-Cyclopentyl-N-(2,5-dimethyl-benzyl)amino]-2-methylamino-purin aus 6-[N-Cyclopentyl-N-(2,5-dimethyl-benzyl)amino]-2-methylamino-9-tetrahydropyran-2-yl-purin | 65 | 203-205 (2-Propanol) |
| n) | 6-[N-Cyclopentyl-N-(furan-2-yl-methyl)amino]-2-methylamino-purin aus Verbindung des Beispiels 23 c | 53 | 162-163 (Diethylether) |
| o) | 6-[N-Cyclopentyl-N-(pyridin-2-yl-methyl)amino]-purin aus Verbindung des Beispiels 2 u | 62 | 200-203 (Diethylether) |

Beispiel 7

6-[N-Benzyl-N-(2-propyl)amino]purin

Eine Loesung aus 3.5 g (9 mmol) 6-[N-Benzyl-N-(2-propyl)amino]-9-ribofuranosyl-purin und 50 ml 2 N Salzsaeure wird 30 min zum Rueckfluß erhitzt. Nach Abkuehlen wird mit Ether gewaschen, die waessrige Phase mit Ammoniakwasser auf pH 7 eingestellt und der Niederschlag aus waessrigem 2-Propanol umkristallisiert. Man erhaelt 1.8 g Titelverbindung (75 % d.Th.) vom Schmp. 200-202° C.

Beispiel 8

In analoger Weise wie in Beispiel 7 beschrieben erhaelt man aus der jeweiligen 9-Ribofuranosyl-Verbindung:

| | Bezeichnung | Ausbeute [%] | Schmelzpunkt °C Lsg.-mittel |
|---|---|---|---|
| a) | 6-[N-Cyclohexyl-N-(2-methyl-propyl)-amino]purin | 72 | 152-153 (2-Propanol) |
| b) | 6-(N-Benzyl-N-cyclopentyl-amino)-purin | 91 | 232-233 (Wasser) |
| c) | 6-[N-Cyclopentyl-N-(4-fluor-benzyl)-amino]purin | 82 | 236-238 (Wasser) |
| d) | 6-[N-Cyclopentyl-N-(furan-2-yl-methyl)-amino]purin | 76 | 181-183 (Diethylether) |
| e) | 6-[N-Cyclopentyl-N-(thiophen-2-yl-methyl)amino]-2-methylamino-purin | 63 | 180-182 (Diethylether) |

Beispiel 8 ( Fortsetzung )

| | Bezeichnung | Ausbeute [%] | Schmelzpunkt°C Lsg.-mittel |
|---|---|---|---|
| f) | 6-[N-Cyclopentyl-N-(thiophen-2-yl-methyl)amino]-2-dimethylamino-purin | 65 | 204-206 (Diethylether) |
| g) | 6-(N-Allyl-N-cyclohexyl-amino)-2-methylamino-purin | 56 | 160-162 (Diethylether) |
| h) | 6-(N-Allyl-N-cyclohexyl-amino)-2-dimethylamino-purin | 69 | 175-177 (Wasser) |
| i) | 6-(N-Allyl-N-cyclohexyl-amino)-8-dimethylamino-purin | 60 | 165-167 (2-Propanol) |
| j) | 6-(N-Allyl-N-cyclohexyl-amino)purin | 94 | 132-133 (Wasser) |
| k) | 6-[N-Cyclopentyl-N-(2,5-dimethyl-benzyl)amino]purin | 79 | 229-231 (Methanol) |
| l) | 6-[N-Cyclopentyl-N-(6-methyl-pyridin-2-yl-methyl)amino]purin | 69 | 113-115 (Wasser) |

Beispiel 9

6-(N-Allyl-N-cyclohexyl-amino)-9-[1-(1,3-dihydroxy-2-propoxy) 2-hydroxy-ethyl]purin

Zu einer Loesung aus 3.9 g (10 mmol) 6-(N-Allyl-N-cyclohexylamino)-9-ribofuranosyl-purin in 100 ml Methanol tropft man bei Raumtemperatur eine Loesung von 2.1 g Natriummetaperiodat in 40 ml Wasser, ruehrt 4 h bei Raumtemperatur, filtriert, engt das Filtrat ein, nimmt in 100 ml Methanol auf, fuegt 20 ml Wasser zu, versetzt mit 3.7 g Natriumtetrahydridoboranat, laeßt 2 h bei Raumtemperatur ruehren, engt ein, nimmt in Dichlormethan auf, waescht mit Wasser, trocknet und engt ein. Man isoliert 2.9 g der Titelverbindung (74 % d.Th.) als amorphes Pulver vom Schmp. 60-65 ° C.

Beispiel 10

In analoger Weise wie in Beispiel 9 beschrieben erhaelt man aus der jeweiligen 9-Ribofuranosylverbindung:

| | Bezeichnung | Ausbeute [%] | Schmelzpunkt°C Lsg.-mittel |
|---|---|---|---|
| a) | 6-[N-Cyclopentyl-N-(2,5-dimethyl-benzyl)-amino]-9-[1-(1,3-dihydroxy-2-propoxy)-2-hydroxy-ethyl]purin | 70 | 80-86 (Dichlormethan) |
| b) | 6-(N-Benzyl-N-cyclopentyl-amino)-9-[1-(1,3-dihydroxy-2-propoxy)-2-hydroxy-ethyl]purin | 72 | 55-60 (Dichlormethan) |
| c) | 6-[N-Cyclopentyl-N-(4-methoxy-benzyl)-amino]-9-[1-(1,3-dihydroxy-2-propoxy)-2-hydroxy-ethyl]purin | 88 | 70-80 (Ethylacetat) |
| d) | 6-[N-Cyclopentyl-N-(thiophen-2-yl-methyl)amino]-9-[1-(1,3-dihydroxy-2-propoxy)-2-hydroxy-ethyl]purin | 62 | 50-55 (Ethylacetat) |

Beispiel 11

6-[N-Benzyl-N-(2-methyl-propyl)amino]-9-(2,3-dihydroxypropyl)purin

Zu einer Mischung aus 1.44 g (30 mmol) 50proz. Natriumhydrid und 100 ml Dimethylformamid tropft man die Loesung von 8.4 g (30 mmol) 6-[N-Benzyl-N-(2-methyl-propyl)amino]purin (Verbindung des Beispiel 5) in 30 ml Dimethylformamid, ruehrt 90 min bei 80° C, tropft die Loesung von 8.6 g (30 mmol) 2,2-Dimethyl-4-(4-methyl-benzolsulfonyloxymethyl)-1,3-dioxolan zu, ruehrt noch 1 h bei 80° C, engt ein, nimmt in Dichlormethan auf, waescht mit Wasser, trocknet und engt ein.

Den Rueckstand nimmt man in 200 ml 0.1 N Salzsaeure auf, ruehrt 8 h bei 80° C, kuehlt ab, waescht mit Ether, stellt alkalisch und extrahiert mit Dichlormethan. Nach Trocknen und Einengen chromatographiert man an Kieselgel (Elutionsmittel Dichlormethan: Methanol 95:5) und isoliert die Titelverbindung als Hydrochlorid. Man erhaelt 5.2 g (44 % d.Th.) vom Schmelzpunkt 155-157° C (aus Diethylether).

Beispiel 12

In analoger Weise wie in Beispiel 11 beschrieben erhaelt man:

| | Bezeichnung | Ausbeute [%] | Schmelzpunkt°C |
|---|---|---|---|
| a) | 6-[N-Cyclopentyl-N-(2,5-dimethyl-benzyl)-amino]-9-(2,3-dihydroxypropyl)purin<br><br>aus Verbindung des Beispiel 8 k | 38 | 70-73<br>(Ligroin) |
| b) | 6-(N-Benzyl-N-cyclopentyl-amino)-9-(2,3-dihydroxypropyl)purin<br>aus Verbindung des Beispiels 8 b | 55 | 133-135<br>(Wasser) |
| c) | 6-[N-Cyclopentyl-N-(4-methoxy-benzyl)-amino]-9-(2,3-dihydroxypropyl)purin<br><br>aus Verbindung des Beispiels 6 k | 42 | 110-112<br>(Diethylether) |
| d) | 6-[N-Cyclopentyl-N-(thiophen-2-yl-methyl)amino]-9-(2,3-dihydroxypropyl)-purin | 33 | 108-110<br>(Diethylether) |
| e) | 6-[N-Cyclohexyl-N-(2-methyl-propyl)-amino]-9-(2,3-dihydroxypropyl)purin<br><br>aus Verbindung des Beispiels 8a | 47 | 60-80<br>amorph<br>(Dichlormethan) |
| f) | 6-[N-Cyclopentyl-N-(3-trifluormethyl-benzyl)amino]-9-(2,3-dihydroxypropyl)-purin<br>aus Verbindung des Beispiel 6 a | 52 | 60-70<br>amorph<br>(Dichlormethan) |
| g) | 6-[N-(5-Chlor-2-methoxy-benzyl)-N-cyclopentyl-amino]-9-(2,3-dihydroxy-propyl)purin<br>aus Verbindung des Beispiel 6 b | 44 | 60-70<br>amorph<br>(Dichlormethan) |

Beispiel 12 ( Fortsetzung)

| | Bezeichnung | Ausbeute [%] | Schmelzpunkt°C Lsg.-mittel |
|---|---|---|---|
| h) | 6-[N-Cyclopentyl-N-(2-methyl-benzyl)-amino]-9-(2,3-dihydroxypropyl)purin<br>aus Verbindung des Beispiel 6 i | 65 | Hydrochlorid 114-116 (Diethylether) |
| i) | 6-[N-Cyclopentyl-N-(4-methyl-benzyl)-amino]-9-(2,3-dihydroxypropyl)purin<br>aus Verbindung des Beispiel 6 c | 45 | 158-160 (Ethylacetat) |
| j) | 6-[N-(Indan-1-yl)-N-(2-methyl-propyl)-amino]-9-(2,3-dihydroxypropyl)purin<br>aus Verbindung des Beispiels 6 f | 29 | Oel (Dichlormethan) |
| k) | 6-(N-Allyl-N-cyclohexyl-amino)-9-(2,3-dihydroxypropyl)purin<br>aus Verbindung des Beispiels 8 j | 47 | Hydrochlorid 70-75 amorph (Diethylether) |
| l) | 6-(N-Allyl-N-cyclohexyl-amino)-9-(2,3-dihydroxypropyl)-2-methylamino-purin<br>aus Verbindung des Beispiels 8 g | 74 | 125-127 (Diethylether) |
| m) | 6-[N-Cyclopentyl-N-(2-methyl-pyridin-6-yl-methyl)amino]-9-(2,3-dihydroxypropyl)purin<br>aus<br>6-[N-Cyclopentyl-N-(2-methyl-pyridin-6-yl-methyl)amino]purin | 54 | 139-140 (Diethylether) |

Beispiel 13

6-(N-Allyl-N-cyclohexyl-amino)-9-(8-methoxycarbonyl-octyl)purin

Zu der Loesung von 0.7 g (30 mmol) Natrium in 100 ml 2-Propanol gibt man 7.7 g (30 mmol) 6-(N-Allyl-N-cyclohexyl-amino)purin (Verbindung des Beispiels 8 j), erwaermt 10 min auf 40° C, traegt 7.5 g (30 mmol) 9-Brom-nonansaeure-methylester ein, erhitzt 16 h zum Rueckfluß, engt ein, nimmt in Wasser auf, extrahiert mit Dichlormethan, trocknet und engt ein.

Nach Chromatographie an Kieselgel (Elutionsmittel Dichlormethan:Methanol 95:5) erhaelt man 4.8 g Titelverbindung (37 % d.Th.) als Oel.

Beispiel 14

6-(N-Allyl-N-cyclohexyl-amino)-9-(8-carboxyoctyl)purin

3.2 g (7.5 mmol) Verbindung des Beispiel 13 werden mit 50 ml 5proz. Natronlauge und 40 ml Ethanol 5 h zum Rueckfluß erhitzt. Man engt ein, nimmt in Wasser auf, waescht mit Ether, stellt die waessrige Phase auf pH 6 ein, extrahiert mit Dichlormethan, trocknet und engt ein. Nach Verreiben mit Diethylether erhaelt man 2.2 g der Titelverbindung (71 % d.Th.) vom Schmp. 100-102 °C.

Beispiel 15

6-(N-Allyl-N-cyclohexyl-amino)-9-(2-hydroxy-propyl)purin

Zu der Loesung von 12.8 g (50 mmol) 6-(N-Allyl-N-cyclohexylamino)purin (Verbindung des Beispiels 8 j) in 150 ml Dimethylsulfoxid gibt man bei 40 °C 7.6 g Kaliumcarbonat, ruehrt 10 min nach, kuehlt auf Raumtemperatur, tropft 5.8 g 1,2-Epoxypropan zu, ruehrt 5 Tage bei Raumtemperatur, engt ein und chromatographiert an Kieselgel (Elutionsmittel Dichlormethan:Methanol 97:3). Nach Verreiben mit Diethylether isoliert man 5.7 g Titelverbindung (36 % d.Th.) vom Schmelzpunkt 108-110 °C.

Beispiel 16

6-(N-Allyl-N-cyclohexyl-amino)-9-(2-acetoxy-propyl)purin

Zu einer Loesung von 2.3 g (7.5 mmol) der Verbindung des Beispiels 15 in 30 ml Pyridin tropft man 1.0 ml Acetanhydrid, ruehrt 16 h bei Raumtemperatur, gießt in Wasser, extrahiert mit Ethylacetat, waescht neutral, trocknet und engt ein. Man erhaelt 1.8 g Titelverbindung (67 % d.Th.) als Oel.

Beispiel 17

6-(N-Allyl-N-cyclohexyl-amino)-9-(2-succinoyloxy-propyl)purin purin

Zu einer Loesung von 3.0 g (10 mmol) Verbindung des Beispiels 15 in 40 ml Pyridin gibt man 1.3 g Bernsteinsaeureanhydrid, erwaermt 3 Tage auf 50 °C, engt ein, nimmt in Dichlormethan auf, waescht mit verd. Essigsaeure, engt ein, nimmt in verd. Natriumhydrogencarbonatloesung auf, waescht mit Diethylether und stellt die waessrige Phase auf pH 5 ein. Man isoliert 2.4 g Titelverbindung (58 % d.Th.) vom Schmp. 122-123 °C.

Beispiel 18

6-[N-Cyclopentyl-N-(2,5-dimethyl-benzyl)amino]-9-(4-cyanobutyl)purin

Zu der Loesung von 0.34 g Natrium in 50 ml 2-Propanol gibt man 4.8 g (15 mmol) Verbindung des Beispiels 8 k, erwaermt 10 min zum Rueckfluß, fuegt 2.45 g 5-Brom-valeronitril zu und kocht 4 Tage am Rueckfluß. Man engt ein, nimmt in Dichlormethan auf, waescht mit Wasser, engt ein und chromatographiert an Kieselgel. Nach Elution mit Dichlormethan:Methanol 97:3 erhaelt man 3.8 g Titelverbindung (63 % d.Th.) als Oel.

Beispiel 19

In analoger Weise wie in Beispiel 18 beschrieben erhaelt man als Oel:

| | Bezeichnung | Ausbeute [%] | |
|---|---|---|---|
| a) | 6-[N-Cyclopentyl-N-(furan-2-yl-methyl)-amino]-9-(4-cyano-butyl)purin aus Verbindung des Beispiel 8d | 57 | |
| b) | 6-[N-Cyclopentyl-N-(thiophen-2-yl-methyl)amino-9-(4-cyano-butyl)purin aus Verbindung des Beispiels 6 j | 46 | |

Beispiel 20

6-[N-Cyclopentyl-N-(2,5-dimethyl-benzyl)amino]-9-[4-(1H-tetrazol-5-yl)butyl]purin-hydrochlorid

Eine Mischung aus 3.8 g (9.4 mmol) Verbindung des Beispiels 18, 1.9 g Natriumazid, 1.5 g Ammoniumchlorid und 30 ml Dimethylformamid wird 3 Tage auf 125° C erhitzt. Man fuegt noch 1.2 g Natriumazid und 1.0 g Ammoniumchlorid zu, ruehrt 6 h bei 125° C nach, kuehlt ab, engt ein, nimmt in Ethylacetat auf, waescht mit Wasser, extrahiert mit 1 N Natronlauge und saeuert die waessrige Phase mit Salzsaeure an. Nach Extraktion mit Dichlormethan, Einengen und verreiben mit Ether erhaelt man 1.7 g Titelverbindung (38 % d.Th.) vom Schmp. 140-142° C.

Beispiel 21

In analoger Weise wie in Beispiel 20 beschrieben erhaelt man:

| | Bezeichnung | Ausbeute [%] | Schmelzpunkt°C Lsg.-mittel |
|---|---|---|---|
| a) | 6-[N-Cyclopentyl-N-(furan-2-yl-methyl)amino]-9-[4-(1H-tetrazol-5-yl)butyl]purin aus Verbindung des Beispiel 19 a | 53 | 113-115 (Diethylether) |
| b) | 6-[N-Cyclopetnyl-N-(thiophen-2-yl-methyl)amino-9-[4-(1H-tetrazol-5-yl)butyl]purin aus Verbindung des Beispiel 19 b | 61 | 109-111 (Diethylether) |

Beispiel 22

6-(N-Allyl-N-oyclohexyl-amino)-2-methylamino-9-tetrahydrofuran-2-yl-purin

Eine Mischung aus 8.5 g (22 mmol) 6-(N-Allyl-N-cyclohexylamino)-2-chlor-9-tetrahydrofuran-2-yl-purin und 150 ml Methanol, das mit Methylamin gesaettigt wurde, wird 48 h im Autoklaven auf 100° C erhitzt. Darauf engt man ein, nimmt in Dichlormethan auf, waescht mit Wasser, trocknet und engt ein. Nach Chromatographie mit Dichlormethan erhaelt man 6.4 g Titelverbindung (82 % d.Th.) als Oel.

Beispiel 23

In analoger Weise wie in Beispiel 22 beschrieben erhaelt man:

| | Bezeichnung | Ausbeute (%) | Schmelzpunkt°C Lsg.-mittel |
|---|---|---|---|
| a) | 6-(N-Allyl-N-cyclohexyl-amino)-2-methylamino-9-tetrahydropyran-2-yl-purin aus 6-(N-Allyl-N-cyclohexyl-amino)-2-chlor-9-tetrahydropyran-2-yl-purin und Methylamin | 67 | Oel (Dichlormethan) |
| b) | 6-[N-Cyclopentyl-N-(3-trifluormethylbenzyl)amino]-2-methylamino-9-tetrahydropyran-2-yl-purin aus 6-[N-Cyclopentyl-N-(3-trifluormethylbenzyl)amino]-2-chlor-9-tetrahydropyran-2-yl-purin und Methylamin | 61 | 133-135 (Diethylether) |
| c) | 6-[N-Cyclopentyl-N-(furan-2-yl-methyl)-amino]-2-methylamino-9-tetrahydropyran-2-yl-purin aus 6-[N-Cyclopentyl-N-(furan-2-yl-methyl)-amino]-2-chlor-9-tetrahydropyran-2-yl-purin und Methylamin | 72 | Oel (Dichlormethan) |

## Ansprüche

1. $N^6$-disubstituierte Purinderivate der Formel I

$$\text{(I)},$$

in welcher

$R_1$ und $R_4$ gleich oder verschieden sind und jeweils wasserstoff, ein Halogenatom oder eine Gruppe $-NR_6R_7$ bedeuten,

$R_2$ und $R_3$ gleich oder verschieden sind und jeweils einen $C_1$- bis $C_6$-Alkylrest, einen $C_2$- bis $C_6$-Alkenylrest, einen $C_3$- bis $C_7$-Cycloalkylrest, einen gewuenschtenfalls am Phenylteil ein- oder zweifach durch Halogen, Hydroxy, $C_1$- bis $C_6$-Alkyl, $C_1$- bis $C_6$-Alkoxy, $C_1$- bis $C_6$-Alkylthio oder Trifluormethyl substituierten geradkettigen oder verzweigten Phenalkylrest mit 1 bis 4 Kohlenstoffatomen im Alkylteil, einen Furylmethyl- oder Furylethylrest, einen Thiophenmethyl- oder Thiophenethylrest, einen gewuenschtenfalls durch einen $C_1$- bis $C_4$-Alkylrest substituierten Pyridylmethylrest oder Pyridylethylrest, einen Indanylrest oder einen Tetrahydronaphthylrest,

$R_5$ Wasserstoff, Tetrahydrofuranyl, Tetrahydropyranyl oder einen $C_1$- bis $C_{10}$-Alkylrest, der gewuenschtenfalls ein- oder mehrfach durch Hydroxy, $C_1$- bis $C_6$-Alkanoyloxy, $C_1$- bis $C_6$-Alkoxycarbonyl, $C_2$- bis $C_6$-Carboxyalkanoyloxy, $C_2$- bis $C_6$-Hydroxyalkoxy, $C_3$-bis $C_6$-Dihydroxyalkoxy, Carboxy, Cyano oder 1H-Tetrazol-5-yl substituiert sein kann,

$R_6$ und $R_7$ die gleich oder verschieden sind, jeweils Wasserstoff, einen $C_1$- bis $C_6$-Alkylrest oder auch gemeinsam einen gewuenschtenfalls durch Sauerstoff, Schwefel oder eine Gruppe $NR_8$ unterbrochenen $C_2$- bis $C_5$-Alkylenrest und

$R_8$ Wasserstoff oder einen $C_1$- bis $C_6$-Alkylrest

bedeuten, mit der Maßgabe, daß

a) $R_2$ und $R_3$ nicht gleichzeitig einen $C_1$-$C_6$-Alkylrest bedeuten können, oder

b) $R_2$ und $R_3$ nicht gleichzeitig ein $C_2$-$C_6$-Alkenylrest, eine unsubstituierte Benzyl- oder Furylmethylgruppe sein können, wenn $R_5$ ein Wasserstoffatom ist, oder

c) $R_2$ nicht eine $C_2$-$C_6$-Alkenylgruppe, eine unsubstituierte Benzyl- oder Furylmethylgruppe sein kann, wenn $R_3$ eine Methylgruppe und $R_5$ ein Wasserstoffatom darstellt,

sowie deren pharmakologisch verträgliche Salze.

**2.** $N^6$-disubstituierte Purinderivate der Formel I gemäß Anspruch 1, in welcher

$R_1$ und $R_4$ gleich oder verschieden sind und jeweils Wasserstoff, eine Methylamino- oder Dimethylamino-Gruppe bedeuten,

$R_2$ und $R_3$ gleich oder verschieden sind und jeweils einen 2-Propyl-, 2-Methylpropyl-, 3-Pentyl-, Allyl-, Cyclopentyl-, Cyclohexyl-, Indanyl-, einen ggf. ein- oder zweifach durch Chlor, Fluor, Methyl, Trifluormethyl oder Methoxy substituierten Benzylrest, eine Furylmethyl-, Thiophenmethyl-, Thiophenethyl-gruppe oder eine ggf. durch Methyl substituierte Pyridinmethylgruppe und

$R_5$ Wasserstoff, Tetrahydrofuranyl, Tetrahydropyranyl oder einen 2,3-Dihydroxypropyl-, 1-(1,3-dihydroxy-2-propoxy)-2-hydroxy-ethyl-, 2-Hydroxpropyl-, 2-Acetoxypropyl-, 2-Succinoyloxy-propyl-, 8-Carboxyoctyl-, 4-Cyanobutyl-, (2-Hydroxyethoxy)methyl oder 4-(1H-tetrazolyl)butylrest

bedeuten, sowie deren pharmakologisch verträgliche Salze.

**3.** $N^6$-disubstituierte Purinderivate gemäß Anspruch 1 oder 2 ausgewählt aus der Gruppe der folgenden Verbindungen:

6-[N-(5-Chlor-2-methoxy)benzyl-N-cyclopentyl-amino]-9-tetrahydro-pyran-2-yl-purin

6-[N-(5-Chlor-2-methoxy)benzyl-N-cyclopentyl-amino]purin

6-[N-Cyclopentyl-N-(2,5-dimethyl-benzyl)-amino]-9-(2,3-dihydroxy-propyl)purin

6-[N-(5-chlor-2-methoxy-benzyl)-N-cyclopentyl-amino]-9-(2,3-dihydroxy-propyl)purin

6-[N-Cyclopentyl-N-(2-methyl-benzyl)-amino]-9-(2,3-dihydroxypropyl)purin

6-[N-Cyclopentyl-N-(2,5-dimethyl-benzyl)amino]-9-tetrahydrofuran-2-yl-purin.

4. Verfahren zur Herstellung von $N^6$-disubstituierten Purinderivaten der Formel I gemäß Auspruch 1,2 oder 3, dadurch gekennzeichnet, daß man in an sich bekannter Weise eine Verbindung der Formel II

(II),

in welcher

$R_1$ und $R_4$ die obengenannte Bedeutung haben,
X einen reaktiven Rest darstellt und
A fuer die obengenannte Gruppe $R_5$ oder einen Ribofuranosylrest steht,
mit einer Verbindung der allgemeinen Formel III

$HNR_2R_3$ (III),

in welcher

$R_2$ und $R_3$ die obengenannte Bedeutung haben, umsetzt

und anschließend gewuenschtenfalls

einen fuer A stehenden Ribofuranosylrest in einen Rest $R_5$ umwandelt oder durch einen Rest $R_5$ ersetzt,

eine in $R_5$ enthaltene Hydroxylgruppe durch Abspaltung einer Schutzgruppe freilegt,

eine in $R_5$ enthaltene Hydroxylgruppe durch Umsetzung mit einem reaktiven Carbonsaeurederivat verestert,

eine in $R_5$ enthaltene Alkoxycarbonylgruppe zur Carboxylgruppe verseift,

eine in $R_5$ enthaltene Cyanogruppe durch Umsetzung mit Stickstoffwasserstoftsaeure in eine 1H-Tetrazol-5-ylgruppe ueberfuehrt,

einen fuer $R_5$ stehenden Tetrahydrofuranyl- oder Tetrahydropyranyl-rest durch Wasserstoff ersetzt,

ein fuer $R_5$ stehendes Wasserstoffatom durch einen anderen, durch die Definition von $R_5$ gegebenen Rest ersetzt,

eine Gruppe $R_1$ oder $R_4$ in eine andere, durch die Definition von $R_1$ und $R_4$ gegebene Gruppe ueberfuehrt,

und die erhaltenen Verbindungen der Formel I gegebenenfalls durch Neutralisation mit nichttoxischen Saeuren in ihre pharmakologisch vertraeglichen Salze umwandelt.

5. Arzneimittel, enthaltend mindestens eine Verbindung gemäß Anspruch 1, 2 oder 3 sowie übliche pharmakologische Träger- und Hilfsstoffe.

6. Verwendung von Verbindungen gemäß Anspruch 1, 2 oder 3 zur Herstellung von Arzneimitteln zur Behandlung allergischer Krankheiten.

## Claims

1. $N^6$-disubstituted purine derivatives of the formula I

(I)

in which $R_1$ and $R_4$ are the same or different and each represents hydrogen, a halogen atom or a group $-NR_6R_7$, $R_2$ and $R_3$ are the same or different and each represents a $C_1$ to $c_6$ alkyl radical, a $C_2$ to $C_4$ alkenyl radical, a $C_3$ to $C_7$ cycloalkyl radical, a straight-chained or branched phenalkyl radical with 1 to 4 carbon atoms in the alkyl moiety substituted, if desired, once or twice by halogen, hydroxyl, $C_1$ to $C_6$ alkyl, $C_1$ to $C_6$ alkoxy, $C_1$ to $C_4$ alkylthio or trifluoromethyl; a furylmethyl or furylethyl radical, a thiophenemethyl or thiopheneethyl radical, a pyridylmethyl or pyridylethyl radical substituted, if desired, by a $C_1$ to $C_4$ alkyl radical, an indanyl radical or a tetrahydronaphthyl radical, $R_5$ signifies hydrogen, tetrahydrofuranyl, tetrahydropyranyl or a $C_1$ to $C_{10}$ alkyl radical which, if desired, can be substituted one or more times by hydroxyl, $C_1$ to $C_6$ alkanoyloxy, $C_1$ to $C_6$ alkoxycarbonyl, $C_2$ to $C_6$ carboxyalkanoyloxy, $C_2$ to $C_6$ hydroxyalkoxy, $C_3$ to $C_6$ dihydroxyalkoxy, carboxyl, cyano or 1H-tetrazol-5-yl, $R_6$ and $R_7$, which are the same or different, each represents hydrogen, a $C_1$ to $C_6$ alkyl radical or also together a $C_2$ to $C_5$ alkylene radical interrupted, if desired, by oxygen, sulphur or a group $NR_8$ and $R_8$ is hydrogen or a $C_1$ to $C_6$ alkyl radical, with the proviso that

a) $R_2$ and $R_3$ cannot simultaneously signify a $C_1$- $C_6$ alkyl radical, or

b) $R_2$ and $R_3$ cannot simultaneously be a $C_2$-$C_6$ alkenyl radical, an unsubstituted benzyl or furylmethyl group when $R_5$ is a hydrogen atom, or

c) $R_2$ cannot be a $C_2$-$C_6$ alkenyl group, an unsubstituted benzyl or furylmethyl group when $R_3$ represents a methyl group and $R_5$ a hydrogen atom,

as well as their pharmacologically acceptable salts.

2. $N^6$-disubstituted purine derivatives of the formula I according to claim 1, in which $R_1$ and $R_4$ are the same or different and each represents hydrogen, a methylamino or dimethylamino group, $R_2$ and $R_3$ are the same or different and each represents a 2-propyl, 2-methylpropyl, 3-pentyl, allyl, cyclopentyl, cyclohexyl, indanyl, a benzyl radical possibly substituted once or twice by chlorine, fluorine, methyl, trifluoromethyl or methoxy; a furylmethyl, thiophenemethyl, thiopheneethyl group or a pyridinemethyl group possibly substituted by methyl and $R_5$ is hydrogen, tetrahydrofuranyl, tetrahydropyranyl or a 2,3-dihydroxypropyl, 1-(1,3-dihydroxy-2-propoxy)-2-hydroxyethyl, 2-hydroxypropyl, 2-acetoxypropyl, 2-succinoylpropyl, 8-carboxyoctyl, 4-cyanobutyl, (2-hydroxyethoxy)-methyl or 4-(1H-tetrazolyl)-butyl radical, as well as their pharmacologically acceptable salts.

3. $N^6$-disubstituted purine derivatives according to claim 1 or 2, selected from the group of the following compounds:

6-[N-(5-chloro-2-methoxy)-benzyl-N-cyclopentylamino]-9-tetrahydropyran-2-ylpurine
6-[N-(5-chloro-2-methoxy)-benzyl-N-cyclopentylamino]-purine
6-[N-cyclopentyl-N-(2,5-dimethylbenzyl)-amino]-9-(2,3-dihydroxypropyl)-purine
6-[N-(5-chloro-2-methoxybenzyl)-N-cyclopentylamino]-9-(2,3-dihydroxypropyl)-purine
6-[N-cyclopentyl-N-(2-methylbenzyl)-amino]-9-(2,3-dihydroxypropyl)-purine
6-[N-cylcopentyl-N-(2,5-dimethylbenzyl)-amino]-9-tetrahydrofuran-2-ylpurine.

**4.** Process for the preparation of $N^6$-disubstituted purine derivatives of the formula I according to claim 1, 2 or 3, characterised in that, in per se known way, one reacts a compound of the formula II

(II)

in which $R_1$ and $R_4$ have the above-given meanings, X represents a reactive residue and A stands for the above-given group $R_5$ or a ribofuranosyl radical, with a compound of the general formula III

$R_2$-NH-$R_3$ (III)

in which $R_2$ and $R_3$ have the above-given meanings, and subsequently, if desired, converts a ribofuranosyl radical standing for A into a radical $R_5$ or replaces by a radical $R_5$, liberates a hydroxyl group contained in $R_5$ by splitting off of a protective group; esterifies a hydroxyl group contained in $R_5$ by reaction with a reactive carboxylic acid derivative; saponifies an alkoxycarbonyl radical contained in $R_5$ to a carboxyl group; converts a cyano group contained in $R_5$ by reaction with hydrazoic acid into a 1H-tetrazol-5-yl group, replaces a tetrahydrofuranyl or tetrahydropyranyl radical standing for $R_5$ by hydrogen, replaces a hydrogen atom standing for $R_5$ by another radical given by the definition of $R_5$; converts a group $R_1$ or $R_4$ into another group given by the definition of $R_1$ and $R_4$; and, if desired, converts the compounds obtained of formula I into their pharmacologically-acceptable salt by neutralisation with non-toxic acids.

**5.** Medicaments containing at least one compound according to claim 1, 2 or 3, as well as usual pharmaceutical carriers and adjuvants.

**6.** The use of compounds according to claim 1, 2 or 3 for the preparation of medicaments for the treatment of allergic diseases.

## Revendications

**1.** Dérivés de purine $N^6$-substitués, de formule I

(I),

dans laquelle

$R_1$ et $R_4$ sont identiques ou différents, et représentent chacun un atome d'hydrogène ou un groupe $-NR_6R_7$,

$R_2$ et $R_3$ sont identiques ou différents, et représentent chacun un groupe alkyle en $C_1$-$C_6$, un groupe alcényle en $C_2$-$C_6$, un groupe cycloalkyle en $C_3$-$C_7$, un groupe phénylalkyle à chaîne droite ou ramifiée, comportant 1 à 4 atomes de carbone dans la partie alkyle, éventuellement une ou deux fois substituée sur la partie phényle par un atome d'halogène, un groupe hydroxyle, un groupe alkyle en $C_1$ - $C_6$, un groupe alcoxy en $C_1$-$C_6$, un groupe alkylthio en $C_1$-$C_6$ ou un groupe trifluorométhyle, un groupe furylméthyle ou un groupe furyléthyle, un groupe thiophèneméthyle ou thiophèneéthyle, un groupe pyridylméthyle ou pyridyléthyle éventuellement substitué par un groupe alkyle en $C_7$-$C_4$, un groupe indanyle ou un groupe tétrahydronaphtyle,

$R_5$ représente un atome d'hydrogène, un groupe tétrahydrofuranyle, un groupe tétrahydropyranyle ou un groupe alkyle en $C_1$-$C_{10}$, qui peut être éventuellement substitué une ou plusieurs fois par un groupe hydroxyle, un groupe alcanoyloxy en $C_1$-$C_6$, un groupe alcoxycarbonyle en $C_1$-$C_6$, un groupe carboxyalcanoyloxy en $C_2$-$C_6$, un groupe hydroxyalcoxy en $C_2$-$C_6$, un groupe dihydroxyalcoxy en $C_3$-$C_6$, un groupe carboxy, cyano ou 1H-tétrazol-5-yle,

$R_6$ et $R_7$ sont identiques ou différents, et représentent chacun un atome d'hydrogène, un groupe alkyle en $C_1$-$C_6$, ou forment conjointement un groupe alkylène en $C_2$-$C_5$, éventuellement interrompu par un atome d'oxygène, un atome de soufre ou un groupe $NR_8$, et

$R_8$ représente un atome d'hydrogène ou un groupe alkyle en $C_1$-$C_6$,

étant entendu que

a) $R_2$ et $R_3$ ne peuvent pas représenter simultanément un groupe alkyle en $C_1$-$C_6$, ou

b) $R_2$ et $R_3$ ne peuvent pas représenter simultanément un groupe alcényle en $C_2$-$C_6$, un groupe benzyle ou furylméthyle non substitué, lorsque $R_5$ représente un atome d'hydrogène, ou

c) $R_2$ ne représente pas un groupe alcényle en $C_2$-$C_6$, un groupe benzyle ou furylméthyle non substitué, lorsque $R_3$ représente un groupe méthyle et $R_5$ représente un atome d'hydrogène,

ainsi que leurs sels pharmacologiquement acceptables.

**2.** Dérivés de purine $N^6$-substitués, de formule I, selon la revendication 1, dans lesquels

$R_1$ et $R_4$ sont identiques ou différents, et représentent chacun un atome d'hydrogène, un groupe méthylamino ou diméthylamino,

$R_2$ et $R_3$ sont identiques ou différents, et représentent chacun un groupe 2-propyle, 2-méthylpropyle, 3-pentyle, allyle, cyclopentyle, cyclohexyle, indanyle, un groupe benzyle éventuellement substitué une ou deux fois par un atome de chlore, de fluor, un groupe méthyle, trifluorométhyle ou méthoxy, un groupe furylméthyle, thiophèneméthyle, thiophèneéthyle ou un groupe pyridineméthyle éventuellement substitué par un groupe méthyle, et

$R_5$ représente un atome d'hydrogène, un groupe tétrahydrofuranyle, un groupe tétrahydropyranyle ou un groupe 2,3-dihydroxypropyle, 1-(1,3-dihydroxy-2-propoxy)-2-hydroxyéthyle, 2-hydroxypropyle, 2-acétoxypropyle, 2-succinoyloxypropyle, 8-carboxyoctyle, 4-cyanobutyle, (2-hydroxyéthoxy)méthyle ou 4-(1H-tétrazolyl)butyle,

ainsi que leurs sels pharmacologiquement acceptables.

**3.** Dérivés de purine $N^6$-substitués, selon la revendication 1 ou 2, choisis dans le groupe constitué par les composés suivants:

6-[N-(5-chloro-2-méthoxy)benzyl-N-cyclopentylamino]-9-tétra-hydropyrane-2-yl-purine

6-[N-(5-chloro-2-méthoxy)benzyl-N-cyclopentylamino]-purine

6-[N-cyclopentyl-N-(2,5-diméthylbenzyl)-amino]-9-(2,3-dihydroxypropyl)-purine

6-[N-(5-chloro-2-méthoxybenzyl)-N-cyclopentylamino]-9-(2,3-dihydroxypropyl)-purine

6-[N-cyclopentyl-N-(2-méthylbenzyl)-amino]-9-(2,3-dihydroxy-propyl)-purine

6-[N-cyclopentyl-N-(2,5-diméthylbenzyl)-amino]-9-tétrahydro-furane-2-yl-purine.

4. Procédé pour la préparation de dérivés de purine $N^6$-substitués, de formule I, selon la revendication 1, 2 ou 3, caractérisé en ce que l'on fait réagir, de manière connue en soi, un composé de formule II

(II),

dans laquelle

| | |
|---|---|
| $R_1$ et $R_4$ | ont les significations mentionnées ci-dessus, |
| X | représente un groupe réactif, et |
| A | représente le groupe $R_5$ mentionné ci-dessus ou un groupe ribofuranosyle, |

avec un composé de formule générale III

$HNR_2R_3$ (III),

dans laquelle

$R_2$ et $R_3$ ont les significations mentionnées ci-dessus, et ensuite, si on le souhaite,
on transforme un groupe ribofuranosyle, représenté par A, en un groupe $R_5$,
on libère un groupe hydroxyle, contenu dans $R_5$, par séparation d'un groupe protecteur,
on estérifie un groupe hydroxyle, contenu dans $R_5$, par réaction avec un dérivé d'acide carboxylique réactif,
on saponifie un groupe alcoxycarbonyle, contenu dans $R_5$, en un groupe carboxyle,
on transforme un groupe cyano, contenu dans $R_5$, par réaction avec l'acide hydroazoïque, en un groupe 1H-tétrazol-5-yle,
on remplace un groupe tétrahydrofuranyle ou tétrahydropyranyle, représenté par $R_5$, par un atome d'hydrogène,
on remplace un atome d'hydrogène, représenté par $R_5$, par un autre groupe selon la définition de $R_5$,
on transforme un groupe $R_1$ ou $R_4$ en un autre groupe selon la définition de $R_1$ et $R_4$,
et l'on transforme éventuellement les composés de formule I obtenus, par neutralisation avec des acides non toxiques, en leurs sels pharmacologiquement acceptables.

5. Médicament contenant au moins un composé selon la revendication 1, 2 ou 3, ainsi que des véhicules et adjuvants usuels.

6. Utilisation des composés selon la revendication 1, 2 ou 3, pour la préparation de médicaments destinés au traitement d'affections allergiques.